# EUROPEAN PATENT SPECIFICATION

(11) **EP 3 459 550 B1**
(45) Date of publication and mention of the grant of the patent: **07.07.2021**
(21) Application number: 16902550.9
(22) Date of filing: 20.05.2016
(51) Int. Cl.: A61K 33/26, A61K 31/355, A61K 31/455, A61K 31/519, A61K 31/714, A61K 31/715, A61K 9/08, A61P 7/06

(54) **INJECTION COMPOSITION COMPRISING AN IRON DEXTRAN COMPLEX AND VITAMINS FOR PREVENTING AND TREATING ANEMIA**
INJEKTIONSZUSAMMENSETZUNG MIT EINEM EISEN-DEXTRAN-KOMPLEX UND VITAMINEN ZUR VORBEUGUNG UND BEHANDLUNG VON ANÄMIE
COMPOSITION POUR INJECTION D'UN COMPLEXE DE FER ET DE DEXTRANE AVEC DES VITAMINES POUR LA PRÉVENTION DES ANÉMIES

(43) Date of publication of application: 27.03.2019
(73) Proprietor: Obshestvo S Ogranichennoj Otvetstvennostju "Vikzdorove Zhivotnyh", Pos. Kraskovo 140051 (RU)
(72) Inventor: CHERNYSHOVA, Marina Pavlovna, Saratov 410012 (RU); SEMENOV, Sergej Vjacheslavovich, Saratov 410019 (RU); PRISTENSKIJ, Dmitrij Vladimirovich, Saratovskaya oblast Ust-Kurdyum 410540 (RU); ANISKOV, Aleksandr Andreevich, Saratov 410031 (RU); VIOLIN, Boris Viktorovich, Moscow 123022 (RU)
(74) Representative: Spengler, Robert
(86) International application number: PCT/RU2016/000301
(87) International publication number: WO 2017/200415

(56) References cited:
- EP-A1- 2 913 054
- US-A- 3 639 588
- US-A1- 2006 134 227
- US-A1- 2007 065 521
- MARKUS R JAHN ET AL: "A comparative study of the physicochemical properties of iron isomaltoside 1000 (Monofer), a new intravenous iron preparation and its clinical implications", EUROPEAN JOURNAL OF PHARMACEUTICS AND BIOPHARMACEUTICS, ELSEVIER SCIENCE PUBLISHERS B.V., AMSTERDAM, NL, vol. 78, no. 3, 15 March 2011 (2011-03-15) , pages 480-491, XP028241966, ISSN: 0939-6411, DOI: 10.1016/J.EJPB.2011.03.016 [retrieved on 2011-03-23]

## Description

### Technical field

The invention relates to the field of medicine and veterinary medicine and can be used to prevent and treat iron deficiency anemia and to promote hematopoiesis.

### Background art

Iron deficiency anemia (IDA) is a pathology caused by the lack of iron in the human body and in mammals. IDA is treated and prevented with preparations of organic or complex iron compounds. Dosages and administration method depend on the diagnosis and extent of IDA. Preparations for oral administration are mainly used to prevent IDA / Pharmacist's Letter/Prescriber's Letter "Comparison of Oral Iron Supplements" August 2008, Vol. 24, Number 240811/. For severe IDA, there are developed and used iron preparations which are iron dextran (ID) complexes / British Pharmacopoea 2009 V. 3 Iron Dextran Injection/, iron complexes with polyisomaltosate, as well as organic salts of iron (saccharate, gluconate, and fumarate) / European J. Pharm. & Biopharm 78 (2011) 480-491. Markus R. Jahn et.al. A comparative study of the physicochemical properties of iron.../. All of these drugs are administered intravenously or intramuscularly.

According to recent data, parenteral formulations, when administered, form iron depots at the injection site and in the liver. Iron-binding transport proteins carry iron into the bone marrow and other hematopoietic organs / Biochim Biophys Acta. 2012 September; 1823(9): 1468-1483. Cole P. Anderson et.al. Mammalian iron metabolism and its control by iron regulatory proteins/.

The high bioavailability of parenteral formulations can quickly achieve high iron concentrations in the blood, but this is just what causes their toxic effects, which manifest themselves as a reduced blood antioxidant status, dysfunctions of the blood organs and liver, and other adverse effects / Drug Res. 1992, 42 (II), 12, 1439-1452. P. Geisser et al. Structure/Histotoxicity relationship of parenteral iron preparations; 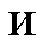 J Anim Sci 1999, 77:1762-1768. G.M. Hill et. al. Effect of vitamin E and selenium on iron utilization in neonatal pigs/.

Currently, no commercial injectable iron supplements are known, which would contain other biologically active substances in amounts providing their therapeutic effect. Some manufacturers produce injectable iron dextran products with vitamins included (as stated). However, the amount and physicochemical stability of these vitamins are insufficient for showing any therapeutic effect.

For example, Suiferrovit-A® (A BIO Ltd., Russian Federation) contains B vitamins as adjuvants with no concentration indicated, Ferrand® (Nita-Farm, Russian Federation) contains folic acid in a concentration of 0.2 mg/ml, what is much lower than the necessary therapeutically effective one.

Unlike injectable iron preparations, those for oral administration, produced in the form of powders, tablets or capsules, contain vitamins and other biologically active substances affecting iron metabolism and the body's antioxidant status.

A composition for the prevention and treatment of IDA is known: US Pat. 8,080,520 "Composition and method for treating iron deficiency anemia". This patent discloses dosage forms of this preparation to treat IDA of the following composition:
- Iron-containing heme (heme iron) or iron polypeptide - from 2 to 12 mg;
- Iron salts or iron chelates - from 15 to 100 mg;
- Folic acid - from 0.1 to 5 mg;
- Vitamin B12 - from 1 to 900 µg.

The preparation is used in the form of tablets and capsules for IDA treatment by oral administration. The patent discloses neither the amounts of adjuvants nor the doses and frequencies of administration. However, the authors claim high efficacy of their product due to synergism of its components, but do not provide specific data on IDA treatment in real patients.

Compositions are known to prevent IDA as powders and tablets containing iron salts with organic acids, vitamins, and other biologically active substances.

These patents offer both medicinal drugs and nutritional supplements in the form of powders (including water-soluble ones), tablets, and capsules for oral administration. Typically, these preparations are used for IDA prophylactics, for example - US Pat. 4,752,479 "Multi vitamin and mineral dietary supplement with controlled release bioavailable iron", EP2330924 "Iron-containing nutritional supplement", and EP0947199 "Composition comprising micro-encapsulated iron".

Other patents claim therapeutic action, for example - WO/2014/135170 "Novel formula of iron based nanocomposites for rapid and efficient treatment of iron deficiency anemia", US Pat. 6,214,373 "Nutritional composition for treating inflammatory bowel diseases", and CN103719661 "Oral liquid health product with function of replenishing blood". But recovery occurs only upon prolonged (over 5 days) administration, which is inadmissible at progressive anemia.

Patent WO/2014/135170 "Novel formula of iron based nanocomposites for rapid and efficient treatment of iron deficiency anemia" offers a new nanocomposite form of iron as powder and liquid for oral administration. The authors state that their drug normalizes blood counts within 3-4 weeks after administering a single dose equivalent to 25 mg of elemental iron, which is contrary to modern data: "A.I. Vorobyov. Guide on hematology. Moscow, Meditsina, 1985" and "L.I. Dvoretski. Iron deficiency anemia. Moscow, Newdiamed, 1998"; in addition, in the amended version of their patent, the authors have removed the claim stating a liquid for oral administration.

In addition, oral administration of iron supplements is known to be ineffective at some diseases violating iron absorption in the alimentary canal. This sharply decreases iron bioavailability and increases the time of treatment and doses (Pharmacist's Letter/Prescriber's Letter "Comparison of Oral Iron Supplements" August 2008, Vol. 24, Number 240811).

Commercial injectable preparations are known for treatment of IDA based on an iron-dextran (ID) complex - Dexferrum® (Vifor Inc., Switzerland); CosmoFer® (Pharmacosmos, Denmark) with an iron (Fe³⁺) content of 50 and 100 mg/mL; Uniferon 200® for animals with an iron (Fe³⁺) content of 200 mg/ml (Pharmacosmos, Denmark); Ursoferran 200® for animals with an iron (Fe³⁺) content of 200 mg/ml (Serumwerk AG, Germany), and similar products of other companies.

Commercial injectable drugs are known based on iron complexes of other poly/oligosaccharide - Monofer 1000®, iron isooligomaltoside, an iron (Fe³⁺) content of 100 mg/ml (Pharmacosmos, Denmark); and Ferinject®, iron carboxymaltoside, an iron (Fe³⁺) content of 50 mg/ml (Vifor, Munchen, Germany).

Iron complexes with dextran and its derivatives were actively developed and patented in the 1950-80s, e.g. US Pat. 3,093,545 "Therapeutic iron dextran preparations", US Pat. RE24642 "Therapeutic preparations of iron", and US Pat. 3,536,696 "Ferric hydroxide dextran and dextrin heptonic acid", except the company Pharmacosmos, which patented its drugs in 1998-2005 (US Pat. 6,977,249 "Process for producing an iron-dextran compound, iron-dextran compound produced according to said process, pharmaceutical composition for prophylaxis or treatment of iron-deficiency and use of said compound for the preparation of parenterally administrable pharmaceutical composition").

All of these preparations represent aqueous solutions of iron-polysaccharide complexes, with no biologically active substances (vitamins and coenzymes) to promote iron assimilation from its complex and to reduce toxicity.

Thus, our analysis of the literature data and patent databases has shown no developments of liquid injectable dosage iron forms with vitamins in amounts to provide their therapeutic effect intended for the treatment of anemia and hematopoiesis promotion.

The preparation Ursoferran 100 and 200® for animals, the iron (Fe³⁺) content of 100 and 200 mg/mL (Serumwerk AG, Germany) is the closest to our proposed solution. This injectable composition to prevent and treat iron deficiency anemia has a complex compound of iron with dextran as the active substance, a preservative, and water.

Its disadvantage is the lack of biologically active substances (vitamins), which entails an increased toxicity and a lower degree of iron assimilation from the complex (just for this, the manufacturers offer products with high iron concentrations).

### Disclosure of the invention

The object of the present invention is to design an injectable medicinal preparation to prevent and treat iron deficiency anemia on the basis of an iron oxide/hydroxide molecular complex with dextran (iron-dextran, ID) containing biologically active substances (vitamins), which would retain their activity and physicochemical stability for a long time (at least 12 months of storage at room temperature) and provide promotion of iron assimilation and hematopoiesis, as well as antioxidant protection of the body.

The technical result is the high efficiency of this drug in IDA treatment, due to the complex therapeutic action of its ingredients and low toxicity as compared to the known products for IDA treatment, by increasing iron assimilation from the complex and hematopoiesis promotion.

Obviously, addition of biologically active substances (vitamins) will lead to a significant improvement of the pharmacological properties of the injectable preparation on the basis of an iron complex by activating hematopoiesis.

All attempts of introducing biologically active substances into products containing any iron complex with derivatives of dextran were unsuccessful due to the rapid destruction of the molecules of biologically active substances in the presence of an iron-polysaccharide complex.

We hypothesize that the degradation of bioactive substances occurs due to a high residual content of free Fe³⁺ ions in the complex solution.

Our solution of the problem to achieve the technical result is based on such an experimentally established fact that at combination of a vitamin, such as folic acid, with ID complexes containing free iron ions lower than or equal to 0.05% the injectable form remains physicochemically stable and the vitamin concentration therein is not reduced. Data on the physicochemical stability of vitamins (folic acid) in commercial ID formulations are shown in Table 1. The preparations were purified from free Fe³⁺ ions by ultrafiltration through a filter with a cutoff of 1,000-2,000 Da (UF purification), followed by adjusting the concentration of the ID complex to 100 mg Fe per ml.

As an active substance, either iron (III) - dextranheptonic acid or iron (III) - oligomaltoside is chosen. For example, isomaltoside 1000 is an oligosaccharide with a mean molecular weight of 1,000 Da, which consists predominantly of chains corresponding to 3-5 glucose units).

The composition contains a solvent, which is water, or an organic solvent, preferably propylene glycol or their mixture.

The composition further comprises, wt%: a preservative - 0.1-2.0; a pH adjuster to pH 6-8.

The composition additionally contains vitamin B12 - cyanocobalamin or hydroxycobalamin, in an amount from 0.001 to 0.1 wt%.

The composition additionally comprises Vitamin E - tocopheryl acetate or α-tocopherol in an amount of 0.1 to 5.0 wt.%, together with a solubilizer, for example, PEG-660-12-hydroxystearate in an amount of 5.0 to 25 wt.%.

The composition can be supplemented with nicotinamide or niacin in an amount of 0.1 to 10 wt. %.

**Table 1. Dependence of vitamin degradation (with folic acid as an example) in few commercial ID preparations on the concentration of free Fe³⁺ ions at an exposure of 30, 60, 180 and 360 days within 20-25°C. The initial concentration of folic acid is 5 mg/mL*.**

| Name/manufacturer of ID complex | Concentration of free Fe³⁺ ions, mg/mL** | Residual concentration of folic acid (mg/ml)*** at exposures, days | | | | |
|---|---|---|---|---|---|---|
| | | 0 | 30 | 60 | 180 | 360 |
| Monofer 1000/ Pharmacosmos | Source/3.10 | 5,31 | 4,63 | 2,12 | 0,10 | 0,00 |
| | UF purification I/1.10 | 5,40 | 4,37 | 3,02 | 2,14 | 1,75 |
| | UF purification II/0.77 | 5,40 | 4,97 | 4,42 | 4,04 | 3,95 |
| CosmoFer/Pharmacosmos | Source/2.18 | 5.14 | 4.87 | 2.88 | 0.09 | 0.00 |
| | UF purification /0.55 | 5.05 | 5.04 | 4.91 | 4.57 | 4.36 |
| Uniferon/Pharmacosmos | Source/3.92 | 4.98 | 4.05 | 1.24 | 0.02 | 0.00 |
| | UF purification /0.63 | 5.11 | 5.00 | 4.92 | 4.03 | 3.18 |
| Ursoferran/Serumwerk AG | Source/1.84 | 5.33 | 5.01 | 2.55 | 1.06 | 0.00 |
| | UF purification /0.41 | 5.21 | 5.20 | 5.20 | 5.00 | 4.92 |

| | | | | | | |
|---|---|---|---|---|---|---|
| * before adding to the preparation, folic acid was neutralized with 0.1 M NaOH solution to pH 6.8-7.2. ** the concentration of free Fe³⁺ ions was measured according to the technique from J. Anal. Chem. (1997) 357: 44-49, M.L. Fernandez-de Cordovanius et al. "Solid-phase spectrophotometric microdetermination of iron with ascorbic acid and ferrozine" with changes. *** the residual concentration of folic acid was measured by the technique from EPharm. 6.0 with changes. | | | | | | |

The data presented in Table 1 clearly show a decreased concentration of folic acid when the content of free iron ions is higher than 0.05%, similar processes are also observed when adding other vitamins, such as B12.

Based on these data, we have developed a dosage form, which is a sterile, stable (physicochemically stable) solution of ID complex with biologically active substances to improve iron assimilation, namely, folic acid (vitamin B9), cyanocobalamin or hydroxocobalamin (vitamin B12), and niacin or niacinamide (vitamin B3), and to reduce toxicity due to neutralization of the oxidative action of iron ions-vitamin E (tocopherol acetate or α-tocopherol). Sodium/potassium hydroxide or monoethanolamine or other amines exhibiting alkaline properties can be used as an acidity (pH) adjuster. This composition is suitable for intravenous or intramuscular administration.

Pharmacological parameters (the toxicity and efficacy in treating IDA) of some drug compositions are given below. Piglets aged 1 to 10 days were taken as an object for estimating the efficiency of the presented compositions, because these animals are known to often suffer from IDA. Few embodiments of the preparation with reduced and increased ID complex contents and the base composition with folic acid were preferably taken.

**Table 2. Compositions of several dosage forms of the ID preparation with vitamins to study pharmacological parameters.**

| Component | Dosage form (DF) (contents of components, %) | | | | | |
|---|---|---|---|---|---|---|
| | DF I (basic) | DF II | DF III | DF IV | DF V | Commercial ID product* |
| ID complex (in terms of Fe⁺) | 2.0 | 2.0 | 2.0 | 10.0 | 0.5 | 10.0 |
| Folic acid | 2.0 | 0.5 | 0.5 | 0.1 | 4.0 | - |
| Vitamin B12 | - | 0.001 | 0.01 | 0.0075 | 0.1 | - |
| Nicotinamide | - | - | 5 | 0.1 | 10 | - |
| Vitamin E | - | 0.1 | 1 | 2 | 5 | - |
| Solubilizer | - | 12 | 12 | 15 | 20 | - |
| NaOH, 1% aq. | To pH 6.0-8.0 | | | | | - |
| Preservative | 1.0 | 1.0 | 1.0 | 1.0 | 1.0 | 0.5 (phenol) |
| Water for injections | to 100 | | | | | |

| | | | | | | |
|---|---|---|---|---|---|---|
| * Ursoferran 100® (manufactured by Serum-Werk) was used as a commercial reference product. | | | | | | |

To study the efficacy and safety of DF I-DF V as compared with the commercially available ID product, 6 groups of piglets were formed with 6 animas in each one. On the 3^{rd} day of life each piglet was intramuscularly injected with 1.5 ml of one of the dosage forms of iron (III) -dextrangeptonic acid complex.

Group 1 - DF I; Group 2 - DF II; Group 3 - DF III; Group 4 - DF IV; Group 5 - DF V; Group 6 - control (the commercial product Ursoferran 100®).

The highest average daily weight gain was observed in groups 3 and 4 (510 g), in groups 1 and 2 the additional weight gain ranged from 300 to 400 g, whilst in Group 5 and the control group the weight gain was 280-310 g. No toxic reactions in the animals in the experimental groups were revealed.

Analysis of the data in Table 3 shows that the biochemical parameters of the piglet blood in groups IV and VI (the commercial preparation), with the same ID content in the preparations of 10%, in the case of additional addition of folic acid, vitamin B12, and nicotinamide into the dosage form, a significant (*p* ≤ 0.05) increase of hemoglobin, hematocrit, the average hemoglobin content in red blood cells, the average concentration of hemoglobin in a red blood cell, iron, ferritin occurs in the piglet blood, and there is a tendency (*p* ≥ 0.05) of an increased red blood cell count and the average volume of a cell. DF I and DF II have almost the same indicators. The parameters and numbers of red blood cells are significantly improved upon the administration of DF III, whereas the biochemical indicators in Group 5 were significantly lower than in the control group due to the insufficient ID amount in DF V.

**Table 3. Results of blood tests and the content dynamics of various iron forms in piglets after administration of various ID products.**

| Component | Dosage form (DF) embodiments | | | | | |
|---|---|---|---|---|---|---|
| | DF I (basic) | DF II | DF III | DF IV | DF V | Commercial ID product (VI) |
| Hematocrit (%) 35-45 | 38.62 ±1.23 | 41.19 ±3.18 | 43.34 ±2.98 | 44.67 ±2.06 | 35.72 ±2.74 | 39.07±1.94 |
| Hemoglobin (g/L) 120-180 | 129.36 ±10.08 | 140.32 ±9.02 | 161.22 ±8.26 | 157.73 ±11.63 | 123.18 ±7.42 | 134.51±8.74 |
| Red blood cells (10¹²/L) 5.8-8.4 | 6.24 ±0.46 | 6.17 ±0.50 | 7.02 ±0.46 | 7.10 ±0.42 | 5.92 ±0.38 | 6.43±0.34 |
| Average hemoglobin content in a red blood cell (pg) 27-34 | 25.32 ±1.08 | 31.76 ±2.17 | 33.92 ±2.44 | 33.16 ±2.36 | 24.94 ±1.75 | 27.16±1.88 |
| Average hemoglobin concentration in a red blood cell (g/L) 280-360 | 296.92± 24.14 | 316.82± 19.12 | 344.56± 17.88 | 339.82± 23.59 | 277.41± 20.16 | 308.62±21.04 |
| Average red blood cell volume (µm³) 80-100 | 78.12 ±3.79 | 88.28 ±4.77 | 96.18 ±4.52 | 90.28 ±4.83 | 79.52 ±3.52 | 83.04±3.16 |
| Iron (µmol/L) 11-35 | 14.37 ±0.92 | 20.14 ±1.34 | 28.17 ±1.52 | 34.31 ±1.92 | 12.26 ±0.73 | 24.45±1.06 |
| TIBC (Total iron binding capacity) (µmol/L) 52-70 | 68.39 ±4.73 | 61.72 ±4.12 | 57.88 ±5.02 | 58.09 ±4.48 | 71.16 ±5.88 | 64.17±4.42 |
| UIBC (Unsaturated iron binding capacity) (µmol/L) 23-63 | 56.81 ±5.72 | 49.78 ±3.74 | 40.12 ±3.76 | 37.24 ±3.23 | 62.74 ±4.92 | 50.20±4.93 |
| Ferritin (µg/ml) 20-200 | 42.54 ±3.66 | 70.08 ±5.40 | 96.23 ±5.34 | 105.49 ±7.17 | 37.09 ±3.02 | 48.42±4.14 |
| Transferrin (g/L) 2-3.65 | 3.71 ±0.29 | 2.92 ±0.18 | 2.18 ±0.15 | 2.23 ±0.18 | 3.58 ±0.21 | 3.62±0.32 |
| Transferrin saturation (%) 20-50 | 24.11 ±2.33 | 33.52 ±2.28 | 38.42 ±2.16 | 35.22 ±1.98 | 21.83 ±1.62 | 27.92±2.69 |

It should be noted that DF III is more advantageous from an economic standpoint, since it has less ID, making it cheaper.

The present invention further provides a preparation technology of the compositions and the most significant examples of dosage forms (DF).

The technology of preparation of the compositions is as follows:
A calculated amount of water for injections is placed into a suitable glass container, a weighed sample of folic acid is added, and the solution is titrated with 10% sodium alkali solution to pH of 6.0 to 8.0 under constant stirring. After preparing this solution of folic acid, weighed samples of the remaining components and a mixture of vitamin E with a solubilizer are added under constant stirring. The resulting solution is filtered through a filter with a pore size of 0.22 µm and filled into sterile vials. Preliminary, ultrafiltration of the ID complex solution is carried out through a filter with a molecular weight cutoff of 1,000 to 2,000 Da, which reduces the content of free iron ions down to 0.05% or lower. When mixing the components directly before using, ultrafiltration is optional.

**Table 4. Basic composition of the dosage forms of the preparation and the optimal contents of other components.**

| Component | Amount of the component, % | |
|---|---|---|
| | Basic DF | Optimal DF |
| ID complex, UF purification (in terms of Fe3⁺) | 2.0 | 2.0 |
| Vitamin B9 (folic acid) | 2.0 | 0.5 |
| Vitamin B12 (cyanocobalamin/ hydroxocobalamin) | - | 0.01 |
| Vitamin B3 (nicotinamide/niacin) | - | 2.0 |
| Vitamin E (tocopherol acetate / α-tocopherol) | - | 1.0 |
| Solubilizer (PEG-660-12-hydroxystearate) | - | 12.0 |
| Sodium hydroxide, 1% solution | 4.0-5.0 | 2.0-3.0 |
| Preservative (benzyl alcohol / chlorobutanol / phenol) | 0.5-1.0 | 0.5-1.0 |
| Water for injections | To 100 | To 100 |

Table 5 contains examples justifying the limits of physicochemical stability of several DFs of the preparation. In all compositions, with the exception of composition No. 3, an iron (III) -dextranheptonic acid complex was used.

**Table 5. Examples of formulations (dosage forms) of the claimed preparation.**

| Component | Example No Content of the component, % | | | | | | | | |
|---|---|---|---|---|---|---|---|---|---|
| | I | II | III | IV | V | VI | VII | VIII | IX |
| ID complex, UF purification (in terms of Fe³⁺) | 2.0 | 2.0 | 2.0 IM* | 20.0 | 20.0 | 0.5 | 0.5 | 10.0 | 10.0 |
| Folic acid or folacin | 4.0 | 4.0 | 4.0 | 0.1 | 0.1 | 4.0 | 4.0 | 2.0 | 2.0 |
| Cyanocobalamin | 0.01 | - | 0.01 | 0.001 | - | 0.1 | - | 0.01 | - |
| Hydroxocobalamin | - | 0.01 | - | - | 0.001 | - | 0.1 | - | 0.01 |
| Nicotinamide | 5.0 | - | 5.0 | 0.1 | - | 10.0 | - | 3.0 | - |
| Niacin | - | 5.0 | - | - | 0.1 | - | 10.0 | - | 3.0 |
| Tocopherol acetate | 3.0 | - | 3.0 | 0.1 | - | 5.0 | - | 2.0 | - |
| α-Tocopherol | - | 3.0 | - | - | 0.1 | - | 5.0 | - | 2.0 |
| pH adjuster | 1-2 | 2-4 | 1-2 | 0.5-1 | 0.5-1 | 2-4 | 4-6 | 2-3 | 3-4 |
| PEG-660-12-hydroxystearate (Solutol®) | 16.0 | 18.0 | 19.0 | 5.0 | 5.0 | 25.0 | 25.0 | 14.0 | 15.0 |
| Phenol | - | - | - | - | - | - | - | 0.5 | 0.5 |
| Chlorobutanol | - | - | - | - | - | 0.5 | 0.5 | - | - |
| Benzyl alcohol | 1.0 | 1.0 | 1.0 | 1.0 | 1.0 | - | - | - | - |
| Water for injections | to 100 | to 100 | to 100 | to 100 | to 100 | to 100 | to 100 | to 100 | to 100 |

| | | | | | | | | | |
|---|---|---|---|---|---|---|---|---|---|
| * IM - iron (III) oligomaltoside. | | | | | | | | | |

Compositions I and II. Examples with the highest levels of folic acid (folacin) and various contents of other vitamins. The DFs based on these active ingredients do not differ in their physicochemical stability and pharmacological properties from the optimal DF.

Composition III. The ID complex is replaced by iron (III) - oligomaltoside. This substitution leads to more rapid degradation of the vitamins due to the high dissociation constant of iron in the complex as compared to the ID complex. Nevertheless, the stability of vitamins remains for quite a long time at +4°C.

Compositions IV and V. The examples with the maximum content of the ID complex. In this case, the content of the other bioactive substances is at a minimum due to the instability of the colloidal ID solution. A further increase in the iron concentration would lead to delamination of the solution and vitamin degradation.

Compositions VI and VII. The examples with the minimum content of the ID complex. In these compositions, chlorobutanol is the best preservative. To improve iron assimilation, the maximum amounts of vitamins are added and, although this composition possesses therapeutic effect, further lowering of the iron content is unacceptable, since the pharmacological parameters of the DF sharply reduce.

Compositions VIII and IX. The examples with medium contents of ID and vitamins with phenol as a preservative. The physicochemical stability and pharmacological properties of these DF coincide with the optimal DF by basic parameters.

## Claims

1. An injectable composition for use in preventing and treating iron deficiency anemia, containing an iron oxide/hydroxide complex with dextran as an active ingredient, and a solvent, wherein the composition additionally contains folic acid with the following contents of the components, wt.%:
- oxide/hydroxide iron complex with dextran - 0.5-20.0;
- folic acid - 0.1-4.0; and
- solvent - the rest;
**characterized in that**
the iron oxide/hydroxide complex with dextran comprises a complex of iron (III) and dextranheptonic acid or a complex of iron (III) oligoisomaltoside as an active ingredient,
wherein the composition additionally contains a pH adjuster to pH within 6.0-8.0, and
wherein the iron complex with dextran contains free iron ions lower than or equal to 0.05%.

2. The composition for use according to claim 1, wherein vitamin B12 - cyanocobalamin or hydroxocobalamin, is added in an amount of 0.001 to 0.1 wt.%.

3. The composition for use according to claim 1, wherein vitamin E - tocopherol acetate or α-tocopherol is added in an amount of 0.1 to 5.0 wt%, together with a solubilizer, for example, PEG-660-12-hydroxystearate in an amount of 5.0 to 25 wt.%.

4. The composition for use according to claim 1, wherein nicotinamide or niacin is added in an amount of 0.1 to 10 wt.%.

5. The composition for use according to claim 1, which additionally comprises (wt.%): a pre-servative - 0.1-2.0.

6. The composition for use according to claim 1, wherein water or an organic solvent, preferably propylene glycol, or a mixture thereof is selected as solvent.

## Patentansprüche

1. Injizierbare Zusammensetzung zur Verwendung bei der Prävention und Behandlung von eisenmangelbedingter Anämie, die einen Eisenoxid/Eisenhydroxid-Komplex mit Dextran als Wirkstoff und ein Lösungsmittel enthält, wobei die Zusammensetzung außerdem Folsäure enthält, mit dem folgenden Komponentengehalt in Gew.-%:
- Oxid/Hydroxid-Eisenkomplex mit Dextran - 0,5 bis 20,0;
- Folsäure - 0,1 bis 4,0; und
- Lösungsmittel - der Rest;
**dadurch gekennzeichnet, dass**
der Eisenoxid/Eisenhydroxid-Komplex mit Dextran einen Komplex aus Eisen(III) und Dextranheptonsäure oder einen Komplex aus Eisen(III)oligoisomaltosid als Wirkstoff umfasst,
wobei die Zusammensetzung außerdem ein pH-Wert einstellendes Mittel enthält, um den pH-Wert auf innerhalb von 6,0 bis 8,0 einzustellen, und
wobei der Eisenkomplex mit Dextran freie Eisenionen zu höchstens 0,05 % enthält.

2. Zusammensetzung zur Verwendung nach Anspruch 1, wobei Vitamin-B12-Cyanocobalamin oder -Hydroxocobalamin in einer Menge von 0,001 bis 0,1 Gew.-% zugesetzt wird.

3. Zusammensetzung zur Verwendung nach Anspruch 1, wobei Vitamin-E-Tocopherolacetat oder -α-Tocopherol in einer Menge von 0,1 bis 5,0 Gew.-% zusammen mit einem Lösungsvermittler, z. B. PEG-660-12-Hydroxystearat, in einer Menge von 5,0 bis 25 Gew.-% zugesetzt wird.

4. Zusammensetzung zur Verwendung nach Anspruch 1, wobei Nikotinamid oder Niacin in einer Menge von 0,1 bis 10 Gew.-% zugesetzt wird.

5. Zusammensetzung zur Verwendung nach Anspruch 1, die außerdem umfasst (Gew.-%): ein Konservierungsmittel - 0,1 bis 2,0.

6. Zusammensetzung zur Verwendung nach Anspruch 1, wobei Wasser oder ein organisches Lösungsmittel, vorzugsweise Propylenglykol, oder ein Gemisch daraus als Lösungsmittel ausgewählt wird.

## Revendications

1. Composition injectable pour utilisation dans la prévention et le traitement de l'anémie ferriprive, contenant un complexe oxyde/hydroxyde de fer avec le dextrane en tant que substance active, et un solvant, où la composition contient en outre de l'acide folique avec les teneurs suivantes des composants, en % en poids :
- complexe oxyde/hydroxyde de fer avec du dextrane : 0,5 à 20,0 ;
- acide folique : 0,1 à 4,0 ; et
- solvant : le reste ;
**caractérisé en ce que**
le complexe oxyde/hydroxyde de fer avec du dextrane comprend un complexe de fer (III) et d'acide dextrane-heptonique ou un complexe d'oligo-isomaltoside de fer (III) en tant que substance active,
où la composition contient en outre un agent d'ajustement du pH à un pH de 6,0 à 8,0, et
dans laquelle le complexe de fer avec le dextrane contient des ions de fer libres à une teneur inférieure ou égale à 0,05 %.

2. Composition pour utilisation selon la revendication 1, dans laquelle de la vitamine B12 - cyanocobalamine ou hydroxocobalamine, est ajoutée en une quantité de 0,001 à 0,1 % en poids.

3. Composition pour utilisation selon la revendication 1, dans laquelle de la vitamine E - acétate de tocophérol ou α-tocophérol est ajoutée en une quantité de 0,1 à 5,0% en poids, conjointement avec un solubilisant, par exemple, PEG-660-12-hydroxystéarate en une quantité de 5,0 à 25 % en poids.

4. Composition pour utilisation selon la revendication 1, dans laquelle du nicotinamide ou de la niacine est ajouté en une quantité de 0,1 à 10 % en poids.

5. Composition pour utilisation selon la revendication 1, qui comprend en outre (% en poids) : un conservateur - 0,1 à 2,0.

6. Composition pour utilisation selon la revendication 1, dans laquelle de l'eau ou un solvant organique, de préférence le propylène glycol, ou un mélange de ceux-ci est choisi en tant que solvant.
